(19) Europäisches Patentamt
European Patent Office
Office européen des brevets

(11) Numéro de publication: **0 530 087 B1**

(12) **FASCICULE DE BREVET EUROPEEN**

(45) Date de publication de fascicule du brevet: **03.05.95**

(51) Int. Cl.6: **C07C 275/24**, C07C 275/32, C07C 275/28, C07C 275/26, C07C 335/12, C07C 335/16, A61K 31/17

(21) Numéro de dépôt: **92402321.1**

(22) Date de dépôt: **21.08.92**

# (54) Nouvelles naphtyléthylurées et naphtyléthylthiourées, leur procédé de préparation et les compositions pharmaceutiques qui les contiennent.

(30) Priorité: **23.08.91 FR 9110547**

(43) Date de publication de la demande:
**03.03.93 Bulletin 93/09**

(45) Mention de la délivrance du brevet:
**03.05.95 Bulletin 95/18**

(84) Etats contractants désignés:
**AT BE CH DE DK ES FR GB GR IE IT LI LU NL PT SE**

(56) Documents cités:
**EP-A- 0 344 425**

**TETRAHEDRON vol. 24, no. 9, Mai 1968, pages 3681 - 3696 K.KOTERA ET AL 'Aziridine formation by lithium aluminum hydride reduction of oximes'**

(73) Titulaire: **ADIR ET COMPAGNIE**
**1 rue Carle Hébert**
**F-92415 Courbevoie Cédex (FR)**

(72) Inventeur: **Lesieur, Daniel**
**20 rue de Verdun**
**F-59147 Gondecourt (FR)**
Inventeur: **Yous, Said**
**Lab. de Chimie Synthèse,**
**3 rue du Pr Laguesse**
**F-59045 Lille Cedex (FR)**
Inventeur: **Depruex, Patrick**
**75 rue Nationale**
**F-59280 Armentieres (FR)**
Inventeur: **Adam, Gérard**
**9 Clos du Mesnil,**
**route du Pecq**
**F-78600 Le Mesnil le Roi (FR)**
Inventeur: **Renard, Pierre**
**50 avenue de Villeneuve l'Etang**
**F-78000 Versailles (FR)**
Inventeur: **Pfeiffer, Bruno**
**6 rue Jean Thomas**
**F-95600 Eaubonne (FR)**
Inventeur: **Guardiola-Lemaitre, Béatrice**
**6 rue Edouard Nortier**
**F-92200 Neuilly sur Seine (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**EP 0 530 087 B1**

Rank Xerox (UK) Business Services
(3.10/3.09/3.3.3)

## Description

La présente invention concerne de nouvelles naphtyléthylurées et naphtyléthylthiourées, leur procédé de préparation et les compositions pharmaceutiques qui les contiennent.

On connait de la littérature, notamment du document EP 344 425, qui constitue l'art antérieur le plus proche, des naphtyléthylurées dotées de propriétés antihypercholestérolémiantes.

La demanderesse a maintenant découvert de nouveaux composés possédant la propriété remarquable de se lier de façon intense et spécifique aux récepteurs de la mélatonine.

Ces composés possèdent de nombreuses et intéressantes activités pharmacologiques de par leur caractère agoniste ou antagoniste de la mélatonine.

Outre leur action bénéfique sur les troubles du rythme circadien et du sommeil et les désordres saisonniers, ils possèdent d'intéressantes propriétés pharmacologiques sur le système nerveux central, notamment anxiolytiques, antipsychotiques, analgésiques, sur l'ovulation, la circulation cérébrale et l'immunomodulation.

Plus spécifiquement, la présente invention concerne les composés de formule générale (I) :

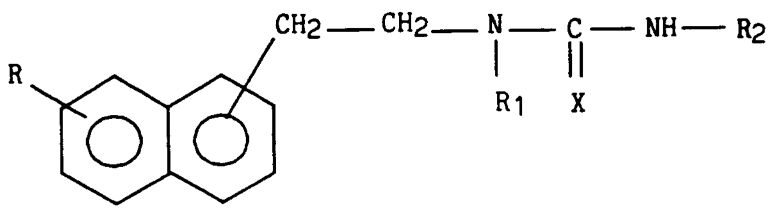

(I)

dans laquelle :

- R représente un atome d'hydrogène ou un groupement $OR_3$ dans lequel $R_3$ représente un atome d'hydrogène, un radical alkyle de 1 à 6 atomes de carbone en chaîne droite ou ramifiée, un radical cycloalkyle ou cycloalkényle contenant de 3 à 8 atomes de carbone, un aryle éventuellement substitué, un arylalkyle ou diarylalkyle éventuellement substitués dans lesquels la chaine alkyle comporte de 1 à 6 atomes de carbone,
- $R_1$ représente un atome d'hydrogène ou un groupe alkyle contenant de 1 à 6 atomes de carbone en chaîne droite ou ramifiée,
- X représente un atome d'oxygène ou de soufre,
- $R_2$ représente un alkyle inférieur de 1 à 6 atomes de carbone linéaire ou ramifié, un cycloalkyle de 3 à 8 atomes de carbone éventuellement substitué, un $(C_3$-$C_8)$cycloalkyl-$(C_1$-$C_6)$alkyle éventuellement substitué, un aryle éventuellement substitué, un arylalkyle éventuellement substitué dont la chaine alkyle comprend de 1 à 6 atomes de carbone, ou un diarylalkyle éventuellement substitué dont la chaine alkyle comprend de 1 à 6 atomes de carbone,

  avec la réserve que lorsque R et $R_1$ représentent un atome d'hydrogène et X représente un atome d'oxygène, $R_2$ ne peut pas représenter un radical phényle ou un radical phényle-2,6 disubstitué.
- leurs sels d'addition à une base pharmaceutiquement acceptable,
- leurs isomères, épimères, diastéréoisomères,
- le terme "substitué" associé aux expressions "aryle", "arylalkyle", "diarylakyle", signifie que le ou les noyaux aromatiques peuvent être substitués par un ou plusieurs groupements choisis parmi alkyle inférieur de 1 à 6 atomes de carbone linéaire ou ramifié, alcoxy inférieur de 1 à 6 atomes de carbone linéaire ou ramifié, hydroxy, halogène, nitro, et trifluorométhyle,
- le terme "substitué" associé aux expressions "cycloalkyle", "cycloalkylalkyle" signifie que le système cyclique peut être substitué par un ou plusieurs groupements choisis parmi halogène, alkyle inférieur de 1 à 6 atomes de carbone linéaire ou ramifié, et alcoxy inférieur de 1 à 6 atomes de carbone linéaire ou ramifié,
- par groupement aryle on entend groupement pyridyle, phényle, naphtyle, thiényle, furyle, pyrimidyle, indolyle, benzofuryle, benzothiényle, ou quinolyle.

La présente invention a également pour objet le procédé de préparation des composés de formule (I), caractérisé en ce que l'on traite une amine de formule générale (II) :

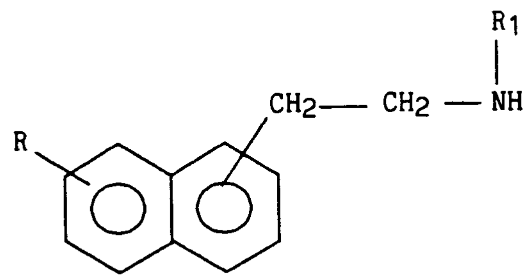

(II)

dans laquelle R et $R_1$ ont les significations ci-dessus définies, par un isocyanate ou un isothiocyanate de formule (III) :

$X = C = N - R_2$ **(III)**

dans laquelle X et $R_2$ ont les mêmes significations que dans la formule (I), de manière à obtenir les composés de formule (I),
composés de formule (I) qui peuvent être, si on le désire,

- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par une base pharmaceutiquement acceptable.

Les composés de formule (I) possèdent des propriétés pharmacologiques intéressantes.

L'étude pharmacologique des composés de l'invention a en effet montré qu'ils étaient peu toxiques, doués d'importantes activités sur le système nerveux central et en particulier, on a relevé des propriétés sédatives, anxiolytiques, antipsychotiques, analgésiques ainsi que sur la microcirculation qui permettent d'établir que les produits de l'invention sont utiles dans le traitement du stress, de l'anxiété, des dépressions saisonnières, des insomnies et fatigues dues aux décalages horaires, des schizophrénies, des attaques de panique, de la mélancolie, des troubles de l'appétit, de l'insomnie, des troubles psychotiques, de l'épilepsie, de la maladie de Parkinson, de la démence sénile, des divers désordres liés au vieillissement normal ou pathologique, des pertes de mémoire, de la maladie d'Alzheimer, ainsi que dans les troubles de la circulation cérébrale.

Dans un autre domaine d'activité, il apparaît que les composés de l'invention possèdent des propriétés d'inhibiteurs de l'ovulation, d'immunomodulateurs et qu'ils sont donc susceptibles d'être utilisés dans le traitement de certains cancers et qu'administrés par voie externe, ils sont utiles dans le traitement du psoriasis, de l'acné, de la séborrhée, protègent la peau et favorisent la pousse des cheveux. Ils peuvent également avoir un usage vétérinaire pour leur propriété sur le pelage.

La présente invention a également pour objet les compositions pharmaceutiques contenant les composés de formule (I) ou le cas échéant un de leurs sels d'addition à une base pharmaceutiquement acceptable, seuls ou en combinaison avec un ou plusieurs excipients ou véhicules inertes, non toxiques, pharmaceutiquement acceptables.

Parmi les compositions pharmaceutiques selon l'invention, on pourra citer, à titre d'exemples et de façon non limitative, celles qui conviennent pour l'administration orale, parentérale, nasale, per/ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, les ampoules buvables et injectables.

La posologie varie selon l'âge et le poids du patient, la voie d'administration, la nature de l'indication thérapeutique, ou des traitements éventuellement associés et s'échelonne entre 0,1 mg et 1 gramme par 24 heures.

Les exemples suivants illustrent l'invention, mais ne la limitent en aucune façon.

Les isocyanates et isothiocyanates de formule (III) sont commerciaux ou peuvent être préparés facilement selon les procédés connus de l'homme de l'art, comme l'action du phosgène ou du thiophosgène sur les amines primaires correspondantes. Les amines de formule (II) peuvent être préparées selon le procédé décrit pour les préparations suivantes, ou selon un procédé équivalent.

**PREPARATION 1 : 2-(7-METHOXY NAPHT-1-YL)ETHYLAMINE**

**STADE A : (7-METHOXY 1,2,3,4-TETRAHYDRO-1-NAPHTYLIDENE) ACETATE D'ETHYLE**

Mélanger 50 g de 7-méthoxy tétralone, 40 g de bromoacétate d'éthyle et 150 $cm^3$ de benzène par l'intermédiaire d'une ampoule à brome. Ajouter le mélange sur du zinc en aiguilles activé (18,6 g) selon Reformatsky et un cristal d'iode.Chauffer à 60 ° C et ensuite porter à reflux pendant 45 minutes.
Hydrolyser sur de la glace en présence d'acide chlorhydrique. Extraire au benzène, sécher et porter à ébullition en présence de $P_2 0_5$. Filtrer et porter à sec.
Le résidu est utilisé tel quel dans l'étape suivante.
Rendement : 80%

**STADE B : (7-METHOXY NAPHT-1-YL) ACETATE D'ETHYLE**

Mélanger 50 g de 7-méthoxy 1,2,3,4 tétrahydro-1-naphtylidène acétate d'éthyle à 7,35 g de soufre et chauffer à 215 ° C pendant 10 heures. Refroidir, ajouter 300 $cm^3$ d'acétate d'éthyle, agiter pendant 30 minutes, filtrer. Porter à sec.
Le résidu obtenu est utilisé tel quel pour l'étape de saponification.
Rendement : 70%

**STADE C : ACIDE (7-METHOXY NAPHT-1-YL) ACETIOUE**

Chauffer à reflux pendant 3 heures un mélange du 7-méthoxy napht-1-yl acétate d'éthyle obtenu précedemment dans 250 cm3 de soude à 20 % dans l'éthanol.
Porter à sec et laver le résidu à l'éther. Précipiter par un courant d'acide chlorhydrique gazeux.
Point de fusion : 155-156 ° C
Rendement : 68%

**STADE D : CHLORURE DE L'ACIDE (7-METHOXY NAPHT-1-YL) ACETIOUE**

Solubiliser à chaud l'acide 7-méthoxy napht-1-yl acétique obtenu précedemment dans 300 $cm^3$ de chloroforme. Chauffer à reflux puis ajouter goutte à goutte le chlorure de thionyle. Porter deux heures à reflux et évaporer à sec. On obtient une huile qui cristallise par refroidissement. Le résidu obtenu est utilisé tel quel dans le stade suivant.

**STADE E : (7-METHOXY NAPHT-1-YL) ACETAMIDE**

Le chlorure de l'acide (7-méthoxy napht-1-yl) acétique obtenu précédemment est dissous dans 200 $cm^3$ d'éther anhydre. Après refroidissement de la solution à l'aide d'un bain de glace-sel, ajouter sous agitation 200 $cm^3$ d'une solution aqueuse d'ammoniaque concentrée.
Agiter 30 minutes et essorer le précipité formé.
Recristalliser dans l'éthanol.
Rendement : 95%
Point de fusion : 201-202 ° C

**STADE F : (7-METHOXY NAPHT-1-YL) ACETONITRILE**

Mettre en suspension le (7-méthoxy napht-1-yl) acétamide obtenu au stade E dans 80 $cm^3$ de tétrahydrofurane anhydre. Ajouter la triéthylamine.
Refroidir la solution dans un bain de glace puis ajouter goutte à goutte l'anhydride trifluoroacétique sous agitation magnétique.
Laisser agiter pendant une heure à température ambiante. Porter à sec. le résidu est repris par de l'eau. Essorer le précipité formé, sécher et recristalliser dans l'éther isopropylique.
Rendement : 83%
Point de fusion : 82-84 ° C
Caractéristiques spectrales :
Infrarouge : 2240 $cm^{-1}$ CN

**STADE G : 2-(7-METHOXY NAPHT-1-YL) ETHYLAMINE**

Placer dans un autoclave une solution de (7-méthoxy napht-1-yl) acétonitrile dans de l'éthanol saturé par de l'ammoniac. Ajouter du nickel de Raney et de l'hydrogène sous 300 atmosphères.
Agiter à 60 °C pendant une nuit. Filtrer et évaporer le filtrat sous vide. L'huile ainsi obtenue est utilisée telle quelle comme matière première.

**PREPARATION 2 : N-METHYL N[2-(7-METHOXY -NAPHT-1-YL) ETHYL] AMINE**

En procédant comme dans la préparation 1, mais en remplaçant au stade E l'ammoniac par la méthylamine, on obtient le N-méthyl (7-méthoxynapht-1-yl) acétamide qui est hydrogéné en composé du titre par le boranediméthylsulfure dans le tétrahydrofurane.

**PREPARATION 3 : 2-(6-METHOXY NAPHT-1-YL) ETHYLAMINE**

En procédant comme dans la préparation 1, mais en remplaçant au stade A la 7-méthoxy tétralone par la 6-méthoxy tétralone, on obtient le composé du titre.

**PREPARATION 4 : 2-(5-METHOXY NAPHT-1-YL) ETHYLAMINE**

En procédant comme dans la préparation 1, mais en remplaçant au stade A la 7-méthoxy tétralone par la 5-méthoxy tétralone, on obtient le composé du titre.

**PREPARATION 5 : 2-(7-METHOXY NAPHT-2-YL) ETHYLAMINE**

En procédant comme dans la préparation 1, mais en remplaçant au stade A la 7-méthoxy 1-tétralone par la 7-méthoxy 2-tétralone, on obtient le composé du titre.

**PREPARATION 6 : 2-(6-METHOXY NAPHT-2-YL) ETHYLAMINE**

En procédant comme dans la préparation 1, mais en remplaçant au stade A la 7-méthoxy 1-tétralone par la 6-méthoxy 2-tétralone, on obtient le composé du titre.

**EXEMPLE 1 : N-[2-(7-METHOXY NAPHT-1-YL)-ETHYL] N′-PROPYLUREE**

A une suspension de 0,01 mole de chlorhydrate de 2-(7-méthoxy napht-1-yl) éthylamine dans 5 $cm^3$ de pyridine, ajouter goutte à goutte sous agitation magnétique 0,011 mole d'isocyanate de propyle. Agiter 1 heure à une température de 80 °C, puis verser le milieu réactionnel sur de l'eau glacée. Acidifier par une solution d'acide chlorhydrique 1N. Le précipité forme est essoré, lavé à l'eau, séché, puis recristallisé dans un mélange toluène-cyclohexane.

On obtient le composé du titre avec 93 % de rendement.

<u>Point de fusion</u> : 104-105 °C
<u>Caractéristiques spectrales en infra-rouge</u> :
3300 $cm^{-1}$ : νNH (urée)
3040 - 2820 $cm^{-1}$ : νCH (alkyles)
1620 - 1600 $cm^{-1}$ : νCC (aromatiques)

**EXEMPLE 2 : N-[2-(7-METHOXY NAPHT-1-YL)-ETHYL] N′-METHYLUREE**

En remplaçant dans l'exemple 1 l'isocyanate de propyle par l'isocyanate de méthyle, on obtient de la même façon le composé du titre
<u>Caractéristiques spectrales en infra-rouge</u> :
3280 $cm^{-1}$ : νNH (urée)
3060 - 2820 $cm^{-1}$ : νCH (alkyles)

**EXEMPLES 3 à 9 :**

En remplaçant dans l'exemple 1, l'isocyanate de propyle par les isocyanates correspondants, on obtient de la même façon les produits des exemples suivants :

**EXEMPLE 3 : N-[2-(7-METHOXY NAPHT-1-YL)-ETHYL] N′-ETHYLUREE**

**EXEMPLE 4 : N-[2-(7-METHOXY NAPHT-1-YL)-ETHYL] N′-BUTYLUREE**

Point de fusion : 106-107 ° C

**EXEMPLE 5 : N-[2-(7-METHOXY NAPHT-1-YL)-ETHYL] N′-BENZYLUREE**

**EXEMPLE 6 : N-[2-(7-METHOXY NAPHT-1-YL)-ETHYL] N′-(4-CHLOROPHENYLMETHYL)UREE**

**EXEMPLE 7 : N-[2-(7-METHOXY NAPHT-1-YL)-ETHYL] N′-CYCLOPROPYLMETHYL UREE**

**EXEMPLE 8 : N-[2-(7-METHOXY NAPHT-1-YL)-ETHYL] N′-[BIS(4-CHLOROPHENYL)METHYL]UREE**

**EXEMPLE 9 : N-[2-(7-METHOXY NAPHT-1-YL)-ETHYL] N′-CYCLOPROPYLUREE**

**EXEMPLE 10 : N-[2-(7-METHOXY NAPHT-1-YL)-ETHYL] N′-PROPYLTHIOUREE**

En procédant comme dans l'exemple 1, mais en utilisant l'isothiocyanate de propyle, on obtient de la même façon le composé du titre :
Point de fusion : 95-97 ° C
Caractéristiques spectrales en infra-rouge :
3240 $cm^{-1}$ : νNH (thiourée)
3060 - 2800 $cm^{-1}$ : νCH (alkyles)

**EXEMPLES 11 à 14 :**

En remplaçant dans l'exemple 10, l'isothiocyanate de propyle par les isothiocyanates correspondants, on obtient de la même façon les composés des exemples suivants :

**EXEMPLE 11 : N-[2-(7-METHOXY NAPHT-1-YL)-ETHYL] N′-METHYLTHIOUREE**

Point de fusion : 105-107 ° C
Caractéristiques spectrales en infra-rouge :
3200 $cm^{-1}$ : νNH (thiourée)

**EXEMPLE 12 : N-[2-(7-METHOXY NAPHT-1-YL)-ETHYL] N′-ETHYLTHIOUREE**

Point de fusion : 114-115 ° C
Caractéristiques spectrales en infra-rouge :
3205 $cm^{-1}$ : νNH (thiourée)

**EXEMPLE 13 : N-[2-(7-METHOXY NAPHT-1-YL)-ETHYL] N′-BUTYLTHIOUREE**

Point de fusion : 65-68 ° C
Caractéristiques spectrales en infra-rouge :
3240 $cm^{-1}$ : νNH (thiourée)

**EXEMPLE 14 : N-[2-(7-METHOXY NAPHT-1-YL)-ETHYL] N′-ISOPROPYLTHIOUREE**

Caractéristiques spectrales en infra-rouge :
3230 $cm^{-1}$ : νNH (thiourée)

**EXEMPLE 15 : N-METHYL N-[2-(7-METHOXY NAPHT-1-YL)-ETHYL] N′-PROPYLUREE**

En remplaçant dans l'exemple 1, la N 2-(7-méthoxy napht-1-yl)éthylamine par la N-[2-(7-méthoxy napht-1-yl)éthyl] N-méthylamine, on obtient de la même façon le composé du titre :
Caractéristiques spectrales en infra-rouge :
3290 $cm^{-1}$ : νNH (urée)

**EXEMPLE 16 : N-[2-(NAPHT-1-YL) ETHYL] N′-PROPYLUREE**

En remplaçant dans l'exemple 1, la 2-(7-méthoxy napht-1-yl)éthylamine par la 2-(napht-1-yl)éthylamine, on obtient de la même façon le composé du titre :
Caractéristiques spectrales en infra-rouge :
3280 $cm^{-1}$ : νNH (urée)

**EXEMPLE 17 : N-[2-(7-HYDROXY NAPHT-1-YL)-ETHYL] N′-PROPYLUREE**

En remplaçant dans l'exemple 1, la 2-(7-méthoxy napht-1-yl)éthylamine par la 2-(7-hydroxy napht-1-yl)-éthylamine, on obtient de la même façon, après purification par chromatographie sur colonne de silice, le composé du titre :
Caractéristiques spectrales en infra-rouge :
3280 $cm^{-1}$ : νNH (urée)
3350 $cm^{-1}$ : νOH (phénol)

**EXEMPLES 18 à 21 :**

En opérant comme dans l'exemple 1, mais en utilisant l'éther de 1-(2-aminoéthyl) 7-hydroxy naphtyle correspondant, on obtient de la même façon les composés des exemples suivants :

**EXEMPLE 18 : N-[2-(7-ISOPROPYLOXY NAPHT-1-YL)-ETHYL] N′-PROPYLUREE**

**EXEMPLE 19 : N-[2-(7-PHENOXY NAPHT-1-YL)-ETHYL] N′-PROPYLUREE**

**EXEMPLE 20 : N-[2-(7-DIPHENYLMETHOXY NAPHT-1-YL)-ETHYL] N′-PROPYLUREE**

**EXEMPLE 21 : N-[2-(7-CYCLOHEXYLOXY NAPHT-1-YL)-ETHYL] N′-PROPYLUREE**

**EXEMPLE 22 : N-[2-(6-METHOXY NAPHT-1-YL)-ETHYL] N′-PROPYLUREE**

En remplaçant dans l'exemple 1, la 2-(7-méthoxy napht-1-yl)éthylamine par la 2-(6-méthoxy napht-1-yl)-éthylamine, on obtient de la même façon le composé du titre :
Caractéristiques spectrales en infra-rouge :
3300 $cm^{-1}$ : νNH (urée)

**EXEMPLE 23 : N-[2-(5-METHOXY NAPHT-1-YL) ETHYL] N′-PROPYLUREE**

En remplaçant dans l'exemple 1, la 2-(7-méthoxy napht-1-yl)éthylamine par la 2-(5-méthoxy napht-1-yl)-éthylamine, on obtient de la même façon le composé du titre :
Caractéristiques spectrales en infra-rouge :
3300 $cm^{-1}$ : νNH (urée)

**EXEMPLE 24 : N-[2-(7-METHOXY NAPHT-2-YL) ETHYL] N′-PROPYLUREE**

En remplaçant dans l'exemple 1, la 2-(7-méthoxy napht-1-yl)éthylamine par la 2-(7-méthoxy napht-2-yl)-éthylamine, on obtient de la même façon le composé du titre :
Caractéristiques spectrales en infra-rouge :
3290 $cm^{-1}$ : νNH (urée)

**EXEMLE 25 : N-[2-(6-METHOXY NAPHT-2-YL) ETHYL] N'-PROPYLUREE**

En remplaçant dans l'exemple 1, la 2-(7-méthoxy napht-1-yl)éthylamine par la 2-(6-méthoxy napht-2-yl)-éthylamine, on obtient de la même façon le composé du titre :
Caractéristiques spectrales en infra-rouge :
3300 $cm^{-1}$ : νNH (urée)

**EXEMPLE 26 : N-[2-(NAPHT-1-YL)-ETHYL] N'-BUTYLUREE**

En remplaçant dans l'exemple 16 l'isocyanate de propyle par l'isocyanate de butyle, on obtient de la même façon le produit du titre :
Caractéristiques spectrales en infra-rouge :
3275 $cm^{-1}$ : νNH (urée)

**EXEMPLE 27 : TEST DE FIXATION AUX RECEPTEURS DE LA MELATONINE**

La fixation aux récepteurs de la mélatonine des composés de l'invention a été réalisée selon des techniques classiques sur des récepteurs de la pars tuberalis de mouton (Journal of Neuroendocrinology (1989), 1 (1), 1-4).

Les composés de l'invention se lient de manière extrêmement spécifique aux récepteurs de la mélatonine avec, pour les plus affins d'entre eux, une affinité de plus de 100 fois supérieure à celle de la mélatonine elle-même. Les meilleurs ont une constante de dissociation (Kd) de l'ordre de $10^{-13}$ mol. $l^{-1}$ contre $6,3.10^{-11}$ $mol.l^{-1}$ pour la mélatonine elle-même.

**EXEMPLE 28 : POTENTIALISATION DU SOMMEIL INDUIT PAR LES BARBITURIOUES**

50 $mg.kg^{-1}$ de pentobarbital sont injectés par voie intrapéritonéale à des souris (22-25 g). On mesure le temps d'apparition et la durée du sommeil. On admet qu'il y a sommeil lorsque les animaux perdent le réflexe de retournement. Les composés à tester sont administrés par voie intrapéritonéale 30 minutes avant l'injection du barbiturique. Les composés de l'invention augmentent la durée du sommeil induit par le pentobarbital.

**EXEMPLE 29 : ETUDE DE LA TOXICITE AIGUE**

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL50 entraînant la mort de 50 % des animaux, a été évaluée.

La DL50 des produits testés est supérieure à 1500 $mg.kg^{-1}$ pour les composés de l'invention étudiés ce qui indique la faible toxicité des composés de l'invention.

**EXEMPLE 30 : COMPOSITION PHARMACEUTIOUE : COMPRIMES**

Comprimés dosés à 10 mg de N-[2-(7-méthoxy napht-1-yl)-éthyl] N'-propylurée

Formule pour 10 000 comprimés :

| | |
|---|---|
| N[2-(7-méthoxy napht-1-yl)-éthyl] N'-propylurée . . | 100 g |
| Amidon de blé . . . . . . . . . . . . . . . . . . . | 400 g |
| Lactose . . . . . . . . . . . . . . . . . . . . . . | 360 g |
| Stéarate de magnésium . . . . . . . . . . . . . . . | 20 g |
| Silice . . . . . . . . . . . . . . . . . . . . . . . | 10 g |
| Hydroxypropyl cellulose . . . . . . . . . . . . . . | 10 g |

**Revendications**

**Revendications pour les Etats contractants suivants : AT, BE, CH, DE, DK, FR, GB, IE, IT, LI, LU, NL, PT, SE**

**1.** Composés de formule générale I :

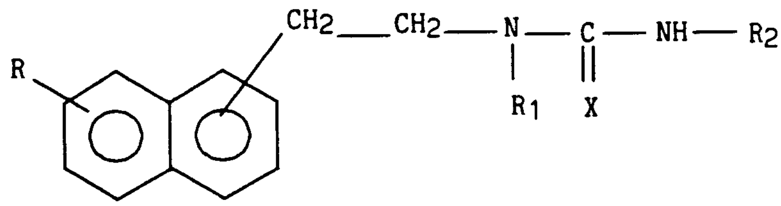

(I)

dans laquelle :

- R représente un atome d'hydrogène ou un groupement $OR_3$ dans lequel $R_3$ représente un atome d'hydrogène, un radical alkyle de 1 à 6 atomes de carbone en chaîne droite ou ramifiée, un radical cycloalkyle ou cycloalkényle contenant de 3 à 8 atomes de carbone, un aryle éventuellement substitué, un arylalkyle ou diarylalkyle éventuellement substitués dans lesquels la chaine alkyle comporte de 1 à 6 atomes de carbone,
- $R_1$ représente un atome d'hydrogène ou un groupe alkyle contenant de 1 à 6 atomes de carbone en chaîne droite ou ramifiée,
- X représente un atome d'oxygène ou de soufre,
- $R_2$ représente un alkyle inférieur de 1 à 6 atomes de carbone linéaire ou ramifié, un cycloalkyle de 3 à 8 atomes de carbone éventuellement substitué, un $(C_3\text{-}C_8)$cycloalkyl-$(C_1\text{-}C_6)$alkyle éventuellement substitué, un aryle éventuellement substitué, un arylalkyle éventuellement substitué dont la chaine alkyle comprend de 1 à 6 atomes de carbone, ou un diarylalkyle éventuellement substitué dont la chaine alkyle comprend de 1 à 6 atomes de carbone, avec la réserve que lorsque R et $R_1$ représentent un atome d'hydrogène et X représente un atome d'oxygène, $R_2$ ne peut pas représenter un radical phényle ou un radical phényle-2,6 disubstitué,
- leurs sels d'addition à une base pharmaceutiquement acceptable,
- leurs isomères, épimères, diastéréoisomères,

étant entendu que :

- le terme "substitué" associé aux expressions "aryle", "arylalkyle", "diarylakyle", signifie que le ou les noyaux aromatiques peuvent être substitués par un ou plusieurs groupements choisis parmi alkyle inférieur de 1 à 6 atomes de carbone linéaire ou ramifié, alcoxy inférieur de 1 à 6 atomes de carbone linéaire ou ramifié, hydroxy, halogène, nitro, et trifluorométhyle,
- le terme "substitué" associé aux expressions "cycloalkyle", "cycloalkylalkyle" signifie que le système cyclique peut être substitué par un ou plusieurs groupements choisis parmi halogène, alkyle inférieur de 1 à 6 atomes de carbone linéaire ou ramifié, et alcoxy inférieur de 1 à 6 atomes de carbone linéaire ou ramifié,
- par groupement aryle on entend groupement pyridyle, phényle, naphtyle, thiényle, furyle, pyrimidyle, indolyle, benzofuryle, benzothiényle, ou quinolyle.

**2.** Composés de formule I selon la revendication 1 pour lesquels R représente un atome d'hydrogène, un radical hydroxyle ou méthoxyle et $R_1$ représente un atome d'hydrogène, leurs sels d'addition à une base pharmaceutiquement acceptable, leurs isomères, épimères, diastéréoisomères.

**3.** Composé de formule I selon la revendication 1 qui est la N-[2-(7-méthoxynapht-1-yl)-éthyl] N'-propylurée.

**4.** Composé de formule I selon la revendication 1 qui est la N-[2-(7-méthoxynapht-1-yl)-éthyl] N'-méthylurée

**5.** Composé de formule I selon la revendication 1 qui est la N-[2-(7-méthoxynapht-1-yl)-éthyl] N'-éthylurée

**6.** Composé de formule I selon la revendication 1 qui est la N-[2-(7-méthoxynapht-1-yl)-éthyl] N'-butylurée

7. Composé de formule I selon la revendication 1 qui est la N-[2-(7-méthoxynapht-1-yl)-éthyl] N'-propylthiourée

8. Composé de formule I selon la revendication 1 qui est la N-[2-(7-méthoxynapht-1-yl)-éthyl] N'-méthylthiourée

9. Composé de formule I selon la revendication 1 qui est la N-[2-(7-méthoxynapht-1-yl)-éthyl] N'-éthylthiourée

10. Composé de formule I selon la revendication 1 qui est la N-[2-(7-méthoxynapht-1-yl)-éthyl] N'-butylthiourée

11. Composé de formule I selon la revendication 1 qui est la N-[2-(napht-1-yl)-éthyl] N'-propylurée

12. Composé de formule I selon la revendication 1 qui est la N-[2-(napht-1-yl)-éthyl] N'-butylurée

13. Procédé de préparation des composés de formule (I) selon la revendication 1, caractérisé en ce que l'on traite une amine de formule générale (II) :

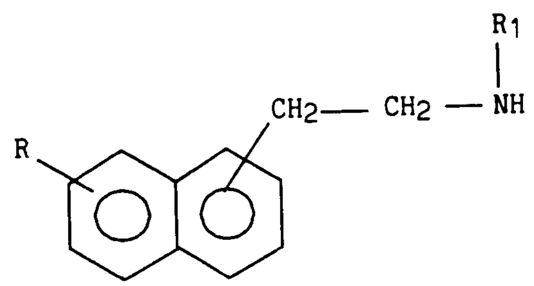

(II)

dans laquelle R et $R_1$ sont tels que définis dans la revendication 1, par un isocyanate ou un isothiocyanate de formule (III) :

$X = C = N - R_2$ **(III)**

dans laquelle X et $R_2$ ont les mêmes significations que dans la revendication 1, de manière à obtenir les composés de formule (I), composés de formule (I) qui peuvent être, si on le désire,

- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés, par une base pharmaceutiquement acceptable.

14. Composition pharmaceutique contenant comme principe actif au moins un composé selon l'une des revendications 1 à 12, seul ou en combinaison avec un ou plusieurs excipients ou véhicules inertes non-toxiques, pharmaceutiquement acceptables.

15. Composition pharmaceutique selon la revendication 14 utile dans le traitement des troubles du système mélatoninergique, du stress, de l'anxiété, des dépressions saisonnières, des insomnies et fatigues dues aux décalages horaires, des schizophrénies, de l'attaque de panique, de la mélancolie, des troubles de l'appétit, de l'insomnie, des troubles psychotiques, de l'épilepsie, de la maladie de Parkinson, de la démence sénile, des divers désordres liés au vieillissement normal ou pathologique, des pertes de mémoire, de la maladie d'Alzheimer, ainsi que des troubles de la circulation cérébrale, de certains cancers, du psoriasis, de l'acné, de la séborrhée ou en tant qu'inhibiteur de l'ovulation ou en médecine vétérinaire dans les troubles du pelage.

**Revendications pour les Etats contractants suivants : ES, GR**

**1.** Procédé de préparation des composés de formule générale I :

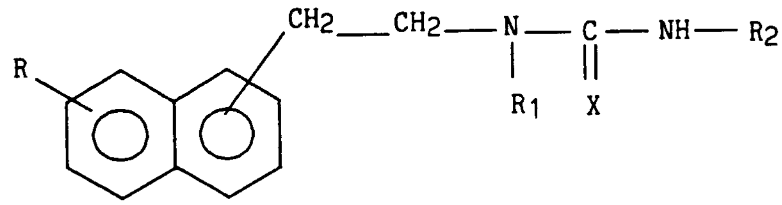

(I)

dans laquelle :

- R représente un atome d'hydrogène ou un groupement $OR_3$ dans lequel $R_3$ représente un atome d'hydrogène, un radical alkyle de 1 à 6 atomes de carbone en chaîne droite ou ramifiée, un radical cycloalkyle ou cycloalkényle contenant de 3 à 8 atomes de carbone, un aryle éventuellement substitué, un arylalkyle ou diarylalkyle éventuellement substitués dans lesquels la chaine alkyle comporte de 1 à 6 atomes de carbone,
- $R_1$ représente un atome d'hydrogène ou un groupe alkyle contenant de 1 à 6 atomes de carbone en chaîne droite ou ramifiée,
- X représente un atome d'oxygène ou de soufre,
- $R_2$ représente un alkyle inférieur de 1 à 6 atomes de carbone linéaire ou ramifié, un cycloalkyle de 3 à 8 atomes de carbone éventuellement substitué, un $(C_3\text{-}C_8)$cycloalkyl-$(C_1\text{-}C_6)$alkyle éventuellement substitué, un aryle éventuellement substitué, un arylalkyle éventuellement substitué dont la chaine alkyle comprend de 1 à 6 atomes de carbone, ou un diarylalkyle éventuellement substitué dont la chaine alkyle comprend de 1 à 6 atomes de carbone,
  avec la réserve que lorsque R et $R_1$ représentent un atome d'hydrogène et X représente un atome d'oxygène, $R_2$ ne peut pas représenter un radical phényle ou un radical phényle-2,6 disubstitué,
- de leurs sels d'addition à une base pharmaceutiquement acceptable,
- de leurs isomères, épimères, diastéréoisomères,

étant entendu que :

- le terme "substitué" associé aux expressions "aryle", "arylalkyle", "diarylakyle", signifie que le ou les noyaux aromatiques peuvent être substitués par un ou plusieurs groupements choisis parmi alkyle inférieur de 1 à 6 atomes de carbone linéaire ou ramifié, alcoxy inférieur de 1 à 6 atomes de carbone linéaire ou ramifié, hydroxy, halogène, nitro, et trifluorométhyle,
- le terme "substitué" associé aux expressions "cycloalkyle", "cycloalkylalkyle" signifie que le système cyclique peut être substitué par un ou plusieurs groupements choisis parmi halogène, alkyle inférieur de 1 à 6 atomes de carbone linéaire ou ramifié, et alcoxy inférieur de 1 à 6 atomes de carbone linéaire ou ramifié,
- par groupement aryle on entend groupement pyridyle, phényle, naphtyle, thiényle, furyle, pyrimidyle, indolyle, benzofuryle, benzothiényle, ou quinolyle,

caractérisé en ce que :

on traite une amine de formule générale (II) :

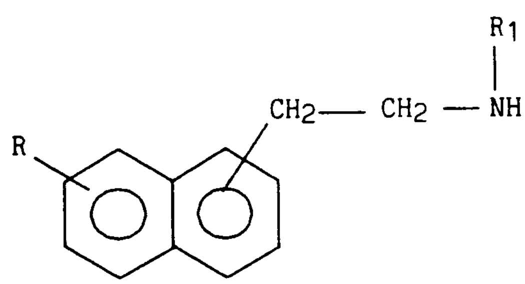

(II)

dans laquelle R et $R_1$ sont tels que définis dans la revendication 1, par un isocyanate ou un

isothiocyanate de formule (III) :

$$X = C = N - R_2 \quad \textbf{(III)}$$

dans laquelle X et $R_2$ ont les mêmes significations que dans la revendication 1, de manière à obtenir les composés de formule (I),
composés de formule (I) qui peuvent être, si on le désire,

- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés, par une base pharmaceutiquement acceptable.

**2.** Procédé de préparation selon la revendication 1 des composés de formule I pour lesquels R représente un atome d'hydrogène, un radical hydroxyle ou méthoxyle et $R_1$ représente un atome d'hydrogène, de leurs sels d'addition à une base pharmaceutiquement acceptable, et de leurs isomères, épimères, et diastéréo-isomères.

**3.** Procédé de préparation selon la revendication 1 du composé de formule I qui est la N-[2-(7-méthoxynapht-1-yl)-éthyl] N′-propylurée.

**4.** Procédé de préparation selon la revendication 1 du composé de formule I qui est la N-[2-(7-méthoxynapht-1-yl)-éthyl] N′-méthylurée

**5.** Procédé de préparation selon la revendication 1 du composé de formule I qui est la N-[2-(7-méthoxynapht-1-yl)-éthyl] N′-éthylurée

**6.** Procédé de préparation selon la revendication 1 du composé de formule I qui est la N-[2-(7-méthoxynapht-1-yl)-éthyl] N′-butylurée

**7.** Procédé de préparation selon la revendication 1 du composé de formule I qui est la N-[2-(7-méthoxynapht-1-yl)-éthyl] N′-propylthiourée

**8.** Procédé de préparation selon la revendication 1 du composé de formule I qui est la N-[2-(7-méthoxynapht-1-yl)-éthyl] N′-méthylthiourée

**9.** Procédé de préparation selon la revendication 1 du composé de formule I qui est la N-[2-(7-méthoxynapht-1-yl)-éthyl] N′-éthylthiourée

**10.** Procédé de préparation selon la revendication 1 du composé de formule I qui est la N-[2-(7-méthoxynapht-1-yl)-éthyl] N′-butylthiourée

**11.** Procédé de préparation selon la revendication 1 du composé de formule I qui est la N-[2-(napht-1-yl)-éthyl] N′-propylurée

**12.** Procédé de préparation selon la revendication 1 du composé de formule I qui est la N-[2-(napht-1-yl)-éthyl] N′-butylurée

**13.** Procédé de préparation d'une composition pharmaceutique contenant comme principe actif au moins un composé préparé selon l'une des revendications 1 à 12, seul ou en combinaison avec un ou plusieurs excipients ou véhicules inertes non-toxiques, pharmaceutiquement acceptables.

**14.** Procédé de préparation selon la revendication 13 d'une composition pharmaceutique utile dans le traitement des troubles du système mélatoninergique, du stress, de l'anxiété, des dépressions saisonnières, des insomnies et fatigues dues aux décalages horaires, des schizophrénies, de l'attaque de panique, de la mélancolie, des troubles de l'appétit, de l'insomnie, des troubles psychotiques, de l'épilepsie, de la maladie de Parkinson, de la démence sénile, des divers désordres liés au vieillissement normal ou pathologique, des pertes de mémoire, de la maladie d'Alzheimer, ainsi que des troubles de la circulation cérébrale, de certains cancers, du psoriasis, de l'acné, de la séborrhée ou en tant qu'inhibiteur de l'ovulation ou en médecine vétérinaire dans les troubles du pelage.

## Claims

**Claims for the following Contracting States : AT, BE, CH, DE, DK, FR, GB, IE, IT, LI, LU, NL, PT, SE**

1. Compounds of the general formula I:

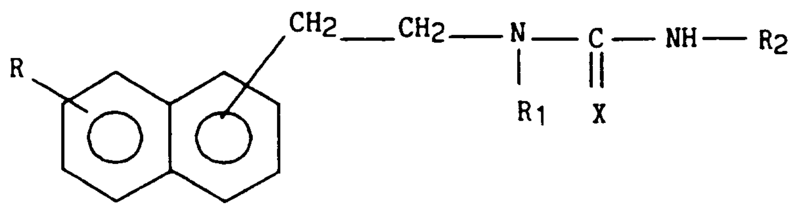

(I)

in which:
- R represents a hydrogen atom or a group $OR_3$ wherein $R_3$ represents a hydrogen atom, a straight-chained or branched alkyl radical having from 1 to 6 carbon atoms, a cycloalkyl or cycloalkenyl radical containing from 3 to 8 carbon atoms, an optionally substituted aryl radical, or an optionally substituted arylalkyl or diarylalkyl radical in which the alkyl chain contains from 1 to 6 carbon atoms;
- $R_1$ represents a hydrogen atom or a straight-chained or branched alkyl group containing from 1 to 6 carbon atoms;
- X represents an oxygen or sulphur atom;
- $R_2$ represents a linear or branched lower alkyl radical having from 1 to 6 carbon atoms, an optionally substituted cycloalkyl radical having from 3 to 8 carbon atoms, an optionally substituted $(C_3-C_8)$-cycloalkyl-$(C_1-C_6)$-alkyl radical, an optionally substituted aryl radical, an optionally substituted arylalkyl radical the alkyl chain of which contains from 1 to 6 carbon atoms, or an optionally substituted diarylalkyl radical the alkyl chain of which contains from 1 to 6 carbon atoms, with the proviso that, when R and $R_1$ each represent a hydrogen atom and X represents an oxygen atom, $R_2$ may not represent a phenyl radical or a 2,6-disubstituted phenyl radical;
- the addition salts thereof with a pharmaceutically acceptable base,
- the isomers, epimers and diastereoisomers thereof,

wherein:
- the term "substituted" associated with the expressions "aryl", "arylalkyl" and "diarylalkyl" indicates that the aromatic nucleus or nuclei may be substituted by one or more groups selected from linear or branched lower alkyl having from 1 to 6 carbon atoms, linear or branched lower alkoxy having from 1 to 6 carbon atoms, hydroxy, halogen, nitro and trifluoromethyl;
- the term "substituted" associated with the expressions "cycloalkyl" and "cycloalkylalkyl" indicates that the cyclic system may be substituted by one or more groups selected from halogen, linear or branched lower alkyl having from 1 to 6 carbon atoms, and linear or branched lower alkoxy having from 1 to 6 carbon atoms, and
- the expression "aryl group" is understood as meaning a pyridyl, phenyl, naphthyl, thienyl, furyl, pyrimidyl, indolyl, benzofuryl, benzothienyl or quinolyl group.

2. Compounds of formula I according to claim 1 in which R represents a hydrogen atom, a hydroxy radical or a methoxy radical and $R_1$ represents a hydrogen atom, the addition salts thereof with a pharmaceutically acceptable base, and the isomers, epimers and diastereoisomers thereof.

3. Compound of formula I according to claim 1 which is N-[2-(7-methoxynaphth-1-yl)ethyl]-N'-propylurea.

4. Compound of formula I according to claim 1 which is N-[2-(7-methoxynaphth-1-yl)ethyl]-N'-methylurea.

5. Compound of formula I according to claim 1 which is N-[2-(7-methoxynaphth-1-yl)ethyl]-N'-ethylurea.

**6.** Compound of formula I according to claim 1 which is N-[2-(7-methoxynaphth-1-yl)ethyl]-N'-butylurea.

**7.** Compound of formula I according to claim 1 which is N-[2-(7-methoxynaphth-1-yl)ethyl]-N'-propylthiourea.

**8.** Compound of formula I according to claim 1 which is N-[2-(7-methoxynaphth-1-yl)ethyl]-N'-methylthiourea.

**9.** Compound of formula I according to claim 1 which is N-[2-(7-methoxynaphth-1-yl)ethyl]-N'-ethylthiourea.

**10.** Compound of formula I according to claim 1 which is N-[2-(7-methoxynaphth-1-yl)ethyl]-N'-butylthiourea.

**11.** Compound of formula I according to claim 1 which is N-[2-(naphth-1-yl)ethyl]-N'-propylurea.

**12.** Compound of formula I according to claim 1 which is N-[2-(naphth-1-yl)ethyl]-N'-butylurea.

**13.** Process for the preparation of the compounds of formula (I) according to claim 1, characterised in that an amine of the general formula (II):

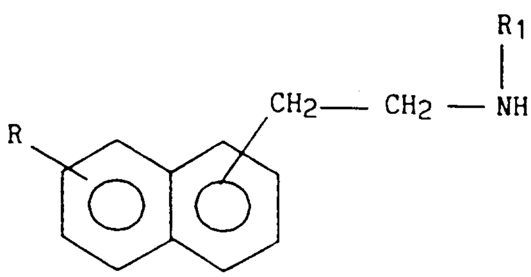

(II),

in which R and $R_1$ are as defined in claim 1, is treated with an isocyanate or isothiocyanate of formula (III):

$X=C=N\text{-}R_2$ (III),

in which X and $R_2$ have the same meanings as in claim 1, to obtain the compounds of formula (I), which compounds of formula (I) may, if desired, be

- purified by one or more purification methods selected from crystallisation, chromatography over a silica column, extraction, filtration, and passage over carbon and/or resin,
- separated, where applicable, in pure form or in the form of a mixture, into their possible optical isomers,
- and/or converted into a salt by means of a pharmaceutically acceptable base.

**14.** Pharmaceutical composition containing as active ingredient at least one compound according to one of claims 1 to 12, on its own or in combination with one or more inert, non-toxic, pharmaceutically acceptable excipients or vehicles.

**15.** Pharmaceutical composition according to claim 14 which can be used in the treatment of disorders of the melatoninergic system, stress, anxiety, seasonal depression, insomnia and fatigue due to jet lag, schizophrenia, panic attacks, melancholia, eating disorders, insomnia, psychotic disorders, epilepsy, Parkinson's disease, senile dementia, various disorders associated with normal or pathological ageing, memory loss and Alzheimer's disease, as well as disorders of cerebral circulation, certain cancers, psoriasis, acne and seborrhoea, or as an ovulation inhibitor, or in veterinary medicine in disorders of the fur.

**Claims for the following Contracting States : ES, GR**

**1.** Process for the preparation of compounds of the general formula I:

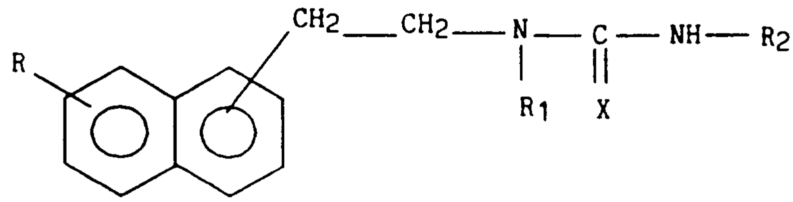

(I)

in which:

- R represents a hydrogen atom or a group $OR_3$ wherein $R_3$ represents a hydrogen atom, a straight-chained or branched alkyl radical having from 1 to 6 carbon atoms, a cycloalkyl or cycloalkenyl radical containing from 3 to 8 carbon atoms, an optionally substituted aryl radical, or an optionally substituted arylalkyl or diarylalkyl radical in which the alkyl chain contains from 1 to 6 carbon atoms;
- $R_1$ represents a hydrogen atom or a straight-chained or branched alkyl group containing from 1 to 6 carbon atoms;
- X represents an oxygen or sulphur atom;
- $R_2$ represents a linear or branched lower alkyl radical having from 1 to 6 carbon atoms, an optionally substituted cycloalkyl radical having from 3 to 8 carbon atoms, an optionally substituted ($C_3$-$C_8$)-cycloalkyl-($C_1$-$C_6$)-alkyl radical, an optionally substituted aryl radical, an optionally substituted arylalkyl radical the alkyl chain of which contains from 1 to 6 carbon atoms, or an optionally substituted diarylalkyl radical the alkyl chain of which contains from 1 to 6 carbon atoms, with the proviso that, when R and $R_1$ each represent a hydrogen atom and X represents an oxygen atom, $R_2$ may not represent a phenyl radical or a 2,6-disubstituted phenyl radical;
- the addition salts thereof with a pharmaceutically acceptable base,
- the isomers, epimers and diastereoisomers thereof,

wherein:

- the term "substituted" associated with the expressions "aryl", "arylalkyl" and "diarylalkyl" indicates that the aromatic nucleus or nuclei may be substituted by one or more groups selected from linear or branched lower alkyl having from 1 to 6 carbon atoms, linear or branched lower alkoxy having from 1 to 6 carbon atoms, hydroxy, halogen, nitro and trifluoromethyl;
- the term "substituted" associated with the expressions "cycloalkyl" and "cycloalkylalkyl" indicates that the cyclic system may be substituted by one or more groups selected from halogen, linear or branched lower alkyl having from 1 to 6 carbon atoms, and linear or branched lower alkoxy having from 1 to 6 carbon atoms, and
- the expression "aryl group" is understood as meaning a pyridyl, phenyl, naphthyl, thienyl, furyl, pyrimidyl, indolyl, benzofuryl, benzothienyl or quinolyl group,

characterised in that an amine of the general formula (II):

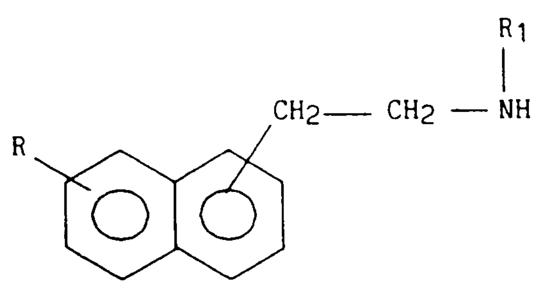

(II),

in which R and $R_1$ are as defined in claim 1, is treated with an isocyanate or isothiocyanate of formula (III):

$X=C=N-R_2$ (III),

in which X and $R_2$ have the same meanings as in claim 1, to obtain the compounds of formula (I), which compounds of formula (I) may, if desired, be

- purified by one or more purification methods selected from crystallisation, chromatography over a silica column, extraction, filtration, and passage over carbon and/or resin,
- separated, where applicable, in pure form or in the form of a mixture, into their possible optical isomers,
- and/or converted into a salt by means of a pharmaceutically acceptable base.

**2.** Process according to claim 1 for the preparation of compounds of formula I in which R represents a hydrogen atom, a hydroxy radical or a methoxy radical and $R_1$ represents a hydrogen atom, the addition salts thereof with a pharmaceutically acceptable base, and the isomers, epimers and diastereoisomers thereof.

**3.** Process according to claim 1 for the preparation of the compound of formula I which is N-[2-(7-methoxynaphth-1-yl)ethyl]-N'-propylurea.

**4.** Process according to claim 1 for the preparation of the compound of formula I which is N-[2-(7-methoxynaphth-1-yl)ethyl]-N'-methylurea.

**5.** Process according to claim 1 for the preparation of the compound of formula I which is N-[2-(7-methoxynaphth-1-yl)ethyl]-N'-ethylurea.

**6.** Process according to claim 1 for the preparation of the compound of formula I which is N-[2-(7-methoxynaphth-1-yl)ethyl]-N'-butylurea.

**7.** Process according to claim 1 for the preparation of the compound of formula I which is N-[2-(7-methoxynaphth-1-yl)ethyl]-N'-propylthiourea.

**8.** Process according to claim 1 for the preparation of the compound of formula I which is N-[2-(7-methoxynaphth-1-yl)ethyl]-N'-methylthiourea.

**9.** Process according to claim 1 for the preparation of the compound of formula I which is N-[2-(7-methoxynaphth-1-yl)ethyl]-N'-ethylthiourea.

**10.** Process according to claim 1 for the preparation of the compound of formula I which is N-[2-(7-methoxynaphth-1-yl)ethyl]-N'-butylthiourea.

**11.** Process according to claim 1 for the preparation of the compound of formula I which is N-[2-(naphth-1-yl)ethyl]-N'-propylurea.

**12.** Process according to claim 1 for the preparation of the compound of formula I which is N-[2-(naphth-1-yl)ethyl]-N'-butylurea.

**13.** Process for the preparation of a pharmaceutical composition containing as active ingredient at least one compound prepared according to one of claims 1 to 12, on its own or in combination with one or more inert, non-toxic, pharmaceutically acceptable excipients or vehicles.

**14.** Process according to claim 13 for the preparation of a pharmaceutical composition which can be used in the treatment of disorders of the melatoninergic system, stress, anxiety, seasonal depression, insomnia and fatigue due to jet lag, schizophrenia, panic attacks, melancholia, eating disorders, insomnia, psychotic disorders, epilepsy, Parkinson's disease, senile dementia, various disorders associated with normal or pathological ageing, memory loss and Alzheimer's disease, as well as disorders of cerebral circulation, certain cancers, psoriasis, acne and seborrhoea, or as an ovulation inhibitor, or in veterinary medicine in disorders of the fur.

**Patentansprüche**

**Patentansprüche für folgende Vertragsstaaten : AT, BE, CH, DE, DK, FR, GB, IE, IT, LI, LU, NL, PT, SE**

1. Verbindungen der allgemeinen Formel I:

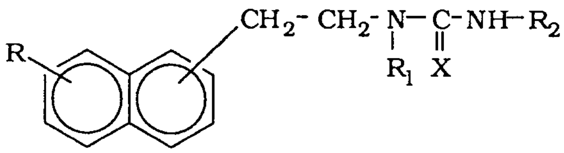

(I)

in der

- R ein Wasserstoffatom oder eine Gruppe $OR_3$, worin $R_3$ ein Wasserstoffatom, eine Alkylgruppe mit 1 bis 6 Kohlenstoffatomen in gerader oder verzweigter Kette, eine Cycloalkyl- oder Cycloalkenylgruppe mit 3 bis 8 Kohlenstoffatomen, eine gegebenenfalls substituierte Arylgruppe, eine gegebenenfalls substituierte Arylalkyl- oder Diarylalkylgruppe, worin die Alkylketten 1 bis 6 Kohlenstoffatome aufweisen, darstellt,
- $R_1$ ein Wasserstoffatom oder eine Alkylgruppe mit 1 bis 6 Kohlenstoffatomen in gerader oder verzweigter Kette,
- X ein Sauerstoff- oder Schwefelatom,
- $R_2$ eine geradkettige oder verzweigte niedrigmolekulare Alkylgruppe mit 1 bis 6 Kohlenstoffatomen, eine gegebenenfalls substituierte Cycloalkylgruppe mit 3 bis 8 Kohlenstoffatomen, eine gegebenenfalls substituierte ($C_3$-$C_8$)-Cycloalkyl-($C_1$-$C_6$)-alkylgruppe, eine gegebenenfalls substituierte Arylgruppe, eine gegebenenfalls substituierte Arylalkylgruppe, deren Alkylkette 1 bis 6 Kohlenstoffatome aufweist, oder eine gegebenenfalls substituierte Diarylalkylgruppe, deren Alkylkette 1 bis 6 Kohlenstoffatome aufweist, mit der Maßgabe bedeuten, daß wenn R und $R_1$ ein Wasserstoffatom und X ein Sauerstoffatom darstellen, $R_2$ nicht eine Phenylgruppe oder eine 2,6-disubstituierte Phenylgruppe bedeutet,
- deren Additionssalze mit einer pharmazeutisch annehmbaren Base,
- deren Isomere, Epimere und Diastereoisomere,

wobei es sich versteht, daß:

- der mit den Ausdrücken "Arylgruppe, "Arylalkylgruppe" und "Diarylalkylgruppe" verknüpfte Begriff "substituiert" bedeutet, daß der oder die aromatischen Kerne durch eine oder mehrere Gruppen ausgewählt aus geradkettigen oder verzweigten niedrigmolekularen Alkylgruppen mit 1 bis 6 Kohlenstoffatomen, geradkettigen oder verzweigten niedrigmolekularen Alkoxygruppen mit 1 bis 6 Kohlenstoffatomen, Hydroxylgruppen, Halogenatomen, Nitrogruppen und Trifluormethylgruppen substituiert sein können,
- der mit den Ausdrücken "Cycloalkylgruppe" und "Cycloalkylalkylgruppe" verknüpfte Begriff "substituiert" bedeutet, daß das cyclische System durch eine oder mehrere Gruppen, ausgewählt aus Halogenatomen, geradkettigen oder verzweigten niedrigmolekularen Alkylgruppen mit 1 bis 6 Kohlenstoffatomen und geradkettigen oder verzweigten niedrigmolekularen Alkoxygruppen mit 1 bis 6 Kohlenstoffatomen substituiert sein kann,
- und unter einer Arylgruppe eine Pyridyl-, Phenyl-, Naphthyl-, Thienyl-, Furyl-, Pyrimidyl-, Indolyl-, Benzofuryl-, Benzothienyl- oder Chinolyl-gruppe zu verstehen ist.

2. Verbindungen der Formel I nach Anspruch 1, worin R ein Wasserstoffatom, eine Hydroxylgruppe oder eine Methoxygruppe und $R_1$ ein Wasserstoffatom bedeuten, sowie deren Additionssalze mit einer pharmazeutisch annehmbaren Base, deren Isomere, Epimere und Diastereoisomere.

3. Verbindung der Formel I nach Anspruch 1, nämlich N-[2-(7-Methoxynaphth-1-yl)-ethyl]-N'-propylharnstoff.

4. Verbindung der Formel I nach Anspruch 1, nämlich N-[2-(7-Methoxynaphth-1-yl)ethyl]-N'-methylharnstoff.

**5.** Verbindung der Formel I nach Anspruch 1, nämlich N-[2-(7-Methoxynaphth-1-yl)-ethyl]-N'-ethylharnstoff.

**6.** Verbindung der Formel I nach Anspruch 1, nämlich N-[2-(7-Methoxynaphth-1-yl)-ethyl]-N'-butylharnstoff.

**7.** Verbindung der Formel I nach Anspruch 1, nämlich N-[2-(7-Methoxynaphth-1-yl)-ethyl]-N'-propylthioharnstoff.

**8.** Verbindung der Formel I nach Anspruch 1, nämlich N-[2-(7-Methoxynaphth-1-yl)-ethyl]-N'-methylthioharnstoff.

**9.** Verbindung der Formel I nach Anspruch 1, nämlich N-[2-(7-Methoxynaphth-1-yl)-ethyl]-N'-ethylthioharnstoff.

**10.** Verbindung der Formel I nach Anspruch 1, nämlich N-[2-(7-Methoxynaphth-1-yl)-ethyl]-N'-butylthioharnstoff.

**11.** Verbindung der Formel I nach Anspruch 1, nämlich N-[2-(Naphth-1-yl)-ethyl]-N'-propylharnstoff.

**12.** Verbindung der Formel I nach Anspruch 1, nämlich N-[2-(Naphth-1-yl)-ethyl]-N'-butylharnstoff.

**13.** Verfahren zur Herstellung der Verbindungen der Formel I nach Anspruch 1, **dadurch gekennzeichnet**, daß man ein Amin der allgemeinen Formel (II):

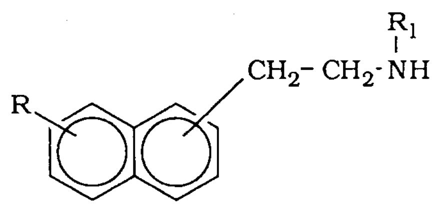

(II)

in der Rund $R_1$ die in Anspruch 1 angegebenen Bedeutungen besitzen, mit einem Isocyanat oder Isothiocyanat der Formel (III):

$X = C = N - R_2$ (III)

worin X und $R_2$ die in Anspruch 1 angegebenen Bedeutungen besitzen, derart behandelt, daß man die Verbindung der Formel (I) erhält, welche Verbindungen der Formel (I) man gewünschtenfalls

- nach einem oder mehreren Reinigungsverfahren ausgewählt aus Kristallisation, Chromatographie über einer mit Siliciumdioxid beschickten Säule, Extraktion, Filtration und Überführung über Kohlenstoff und/oder Harz reinigen kann,
- gegebenenfalls in reiner Form oder in Form einer Mischung in deren eventuell vorhandene optische Isomeren trennen kann,
- und/oder mit einer pharmazeutisch annehmbaren Base in ein Salz überführen kann.

**14.** Pharmazeutische Zubereitung enthaltend als Wirkstoff mindestens eine Verbindung nach einem der Ansprüche 1 bis 12 allein oder in Kombination mit einem oder mehreren inerten, nichttoxischen, pharmazeutisch annehmbaren Trägermaterialien oder Bindemitteln.

**15.** Pharmazeutische Zubereitung nach Anspruch 14 zur Behandlung von Störungen des melatoninergischen Systems, des Streß, der Angst, von jahreszeitlich bedingten Depressionen, der Schlaflosigkeit und der Müdigkeit als Folge von Zeitverschiebungen, der Schizophrenie, von Panikanfällen, der Melancholie, von Appetitstörungen, der Schlaflosigkeit, von psychotischen Störungen, der Epilepsie, der Parkinsonschen Krankheit, des Altersirrsinns, von verschiedenen Störungen, die mit dem normalen oder dem pathologischen Altern verknüpft sind, von Gedächtnisverlusten, der Alzheimerschen Krankheit

sowie von Störungen der Blutzirkulation des Gehirns, von bestimmten Krebsen, der Psoriasis, der Akne, der Seborrhoe oder als Ovulationsinhibitor oder in der Veterinärmedizin bei Störungen des Haarkleids.

**Patentansprüche für folgende Vertragsstaaten : ES, GR**

**1.** Verfahren zur Herstellung der Verbindungen der allgemeinen Formel I:

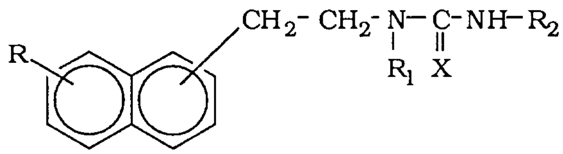

(I)

in der

- R ein Wasserstoffatom oder eine Gruppe $OR_3$, worin $R_3$ ein Wasserstoffatom, eine Alkylgruppe mit 1 bis 6 Kohlenstoffatomen in gerader oder verzweigter Kette, eine Cycloalkyl- oder Cycloalkenylgruppe mit 3 bis 8 Kohlenstoffatomen, eine gegebenenfalls substituierte Arylgruppe, eine gegebenenfalls substituierte Arylalkyl- oder Diarylalkylgruppe, worin die Alkylketten 1 bis 6 Kohlenstoffatome aufweisen, darstellt,
- $R_1$ ein Wasserstoffatom oder eine Alkylgruppe mit 1 bis 6 Kohlenstoffatomen in gerader oder verzweigter Kette,
- X ein Sauerstoff- oder Schwefelatom,
- $R_2$ eine geradkettige oder verzweigte niedrigmolekulare Alkylgruppe mit 1 bis 6 Kohlenstoffatomen, eine gegebenenfalls substituierte Cycloalkylgruppe mit 3 bis 8 Kohlenstoffatomen, eine gegebenenfalls substituierte ($C_3$-$C_8$)-Cycloalkyl-($C_1$-$C_6$)-alkylgruppe, eine gegebenenfalls substituierte Arylgruppe, eine gegebenenfalls substituierte Arylalkylgruppe, deren Alkylkette 1 bis 6 Kohlenstoffatome aufweist, oder eine gegebenenfalls substituierte Diarylalkylgruppe, deren Alkylkette 1 bis 6 Kohlenstoffatome aufweist, mit der Maßgabe bedeuten, daß wenn Rund $R_1$ ein Wasserstoffatom und X ein Sauerstoffatom darstellen, $R_2$ nicht eine Phenylgruppe oder eine 2,6-disubstituierte Phenylgruppe bedeutet,
- von deren Additionssalzen mit einer pharmazeutisch annehmbaren Base,
- von deren Isomeren, Epimeren und Diastereoisomeren,

wobei es sich versteht, daß:

- der mit den Ausdrücken "Arylgruppe, "Arylalkylgruppe" und "Diarylalkylgruppe" verknüpfte Begriff "substituiert" bedeutet, daß der oder die aromatischen Kerne durch eine oder mehrere Gruppen ausgewählt aus geradkettigen oder verzweigten niedrigmolekularen Alkylgruppen mit 1 bis 6 Kohlenstoffatomen, geradkettigen oder verzweigten niedrigmolekularen Alkoxygruppen mit 1 bis 6 Kohlenstoffatomen, Hydroxylgruppen, Halogenatomen, Nitrogruppen und Trifluormethylgruppen substituiert sein können,
- der mit den Ausdrücken "Cycloalkylgruppe" und "Cycloalkylalkylgruppe" verknüpfte Begriff "substituiert" bedeutet, daß das cyclische System durch eine oder mehrere Gruppen, ausgewählt aus Halogenatomen, geradkettigen oder verzweigten niedrigmolekularen Alkylgruppen mit 1 bis 6 Kohlenstoffatomen und geradkettigen oder verzweigten niedrigmolekularen Alkoxygruppen mit 1 bis 6 Kohlenstoffatomen substituiert sein kann,
- und unter einer Arylgruppe eine Pyridyl-, Phenyl-, Naphthyl-, Thienyl-, Furyl-, Pyrimidyl-, Indolyl-, Benzofuryl-, Benzothienyl- oder Chinolyl-gruppe zu verstehen ist,

**dadurch gekennzeichnet**, daß man ein Amin der allgemeinen Formel (II):

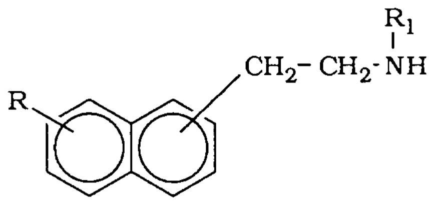

(II)

in der R und $R_1$ die in Anspruch 1 angegebenen Bedeutungen besitzen, mit einem Isocyanat oder Isothiocyanat der Formel (III):

$$X = C = N - R_2 \qquad (III)$$

worin Kund $R_2$ die in Anspruch 1 angegebenen Bedeutungen besitzen, derart behandelt, daß man die Verbindung der Formel (I) erhält, welche Verbindungen der Formel (I) man gewünschtenfalls

- nach einem oder mehreren Reinigungsverfahren ausgewählt aus Kristallisation, Chromatographie über einer mit Siliciumdioxid beschickten Säule, Extraktion, Filtration und Überführung über Kohlenstoff und/oder Harz reinigen kann,
- gegebenenfalls in reiner Form oder in Form einer Mischung in deren eventuell vorhandene optische Isomeren trennen kann,
- und/oder mit einer pharmazeutisch annehmbaren Base in ein Salz überführen kann.

**2.** Verfahren nach Anspruch 1 zur Herstellung von Verbindungen der Formel I, worin R ein Wasserstoffatom, eine Hydroxylgruppe oder eine Methoxygruppe und $R_1$ ein Wasserstoffatom bedeuten, von deren Additionssalzen mit einer pharmazeutisch annehmbaren Base und von deren Isomeren, Epimeren und Diastereoisomeren.

**3.** Verfahren nach Anspruch 1 zur Herstellung der Verbindung der Formel I, nämlich N-[2-(7-Methoxynaphth-1-yl)-ethyl]-N'-propylharnstoff.

**4.** Verfahren nach Anspruch 1 zur Herstellung der Verbindung der Formel I, nämlich N-[2-(7-Methoxynaphth-1-yl)-ethyl]-N'-methylharnstoff.

**5.** Verfahren nach Anspruch 1 zur Herstellung der Verbindung der Formel I, nämlich N-[2-(7-Methoxynaphth-1-yl)-ethyl]-N'-ethylharnstoff.

**6.** Verfahren nach Anspruch 1 zur Herstellung der Verbindung der Formel I, nämlich N-[2-(7-Methoxynaphth-1-yl)-ethyl]-N'-butylharnstoff.

**7.** Verfahren nach Anspruch 1 zur Herstellung der Verbindung der Formel I, nämlich N-[2-(7-Methoxynaphth-1-yl)-ethyl]-N'-propylthioharnstoff.

**8.** Verfahren nach Anspruch 1 zur Herstellung der Verbindung der Formel I, nämlich N-[2-(7-Methoxynaphth-1-yl)-ethyl]-N'-methylthioharnstoff.

**9.** Verfahren nach Anspruch 1 zur Herstellung der Verbindung der Formel I, nämlich N-[2-(7-Methoxynaphth-1-yl)-ethyl]-N'-ethylthioharnstoff.

**10.** Verfahren nach Anspruch 1 zur Herstellung der Verbindung der Formel I, nämlich N-[2-(7-Methoxynaphth-1-yl)-ethyl)-N'-butylthioharnstoff.

**11.** Verfahren nach Anspruch 1 zur Herstellung der Verbindung der Formel I, nämlich N-[2-(Naphth-1-yl)-ethyl]-N'-propylharnstoff.

**12.** Verfahren nach Anspruch 1 zur Herstellung der Verbindung der Formel I, nämlich N-[2-(Naphth-1-yl)-ethyl]-N'-butylharnstoff.

**13.** Verfahren zur Herstellung einer pharmazeutischen Zubereitung enthaltend als Wirkstoff mindestens eine Verbindung hergestellt nach einem der Ansprüche 1 bis 12 allein oder in Kombination mit einem oder mehreren inerten, nichttoxischen, pharmazeutisch annehmbaren Trägermaterialien oder Bindemitteln.

**14.** Verfahren nach Anspruch 13 zur Herstellung einer pharmazeutischen Zubereitung zur Behandlung von Störungen des melatoninergischen Systems, des Streß, der Angst, von jahreszeitlich bedingten Depressionen, der Schlaflosigkeit und der Müdigkeit als Folge von Zeitverschiebungen, der Schizophrenie, von Panikanfällen, der Melancholie, von Appetitstörungen, der Schlaflosigkeit, von psychotischen Störungen, der Epilepsie, der Parkinsonschen Krankheit, des Altersirrsinns, von verschiedenen Störungen, die mit dem normalen oder dem pathologischen Altern verknüpft sind, von Gedächtnisverlusten, der Alzheimerschen Krankheit sowie von Störungen der Blutzirkulation des Gehirns, von bestimmten Krebsen, der Psoriasis, der Akne, der Seborrhoe oder als Ovulationsinhibitor oder in der Veterinärmedizin bei Störungen des Haarkleids.